# EUROPÄISCHE PATENTSCHRIFT

(11) **EP 0 917 463 B1**
(45) Veröffentlichungstag und Bekanntmachung des Hinweises auf die Patenterteilung: **20.02.2002**
(21) Anmeldenummer: 97935532.8
(22) Anmeldetag: 19.07.1997
(51) Int. Cl.: A61K 31/135, A61K 9/52, A61P 29/00

(54) **TRAMADOL MULTIPLE UNIT FORMULIERUNGEN**
TRAMADOL MULTIPLE UNIT FORMULATIONS
FORMULATIONS DE TRAMADOL A UNITES MULTIPLES

(30) Priorität: 25.07.1996 DE 19630035
(43) Veröffentlichungstag der Anmeldung: 26.05.1999
(73) Patentinhaber: ASTA Medica Aktiengesellschaft, 01277 Dresden (DE)
(72) Erfinder: MOMBERGER, Helmut, D-35037 Marburg (DE); RABER, Marc, D-35390 Giessen (DE); KUHN, Dieter, D-35039 Marburg (DE); SCHMID, Wolfgang, D-35102 Lohra (DE)
(74) Vertreter: Wibbelmann, Jobst, Dr., Dipl.-Chem.
(86) Internationale Anmeldenummer: EP9703934
(87) Internationale Veröffentlichungsnummer: WO9804249

(56) Entgegenhaltungen:
- WO-A-95/14460
- US-A- 5 093 200
- US-A- 5 395 626
- US-A- 5 474 786

## Beschreibung

Die Erfindung betrifft perorale Multiple Unit Formulierungen bestehend aus Retardpellets, die Tramadol oder physiologisch verträgliche Salze davon und mindestens einen pharmazeutisch akzeptablen, die Freisetzung verzögernden Stoff enthalten sowie Verfahren zu ihrer Herstellung.

Tramadol((1RS;2RS)-2[(Dimethylamino)methyl]-1-(3-methoxyphenyl)-cyclohexanol)ist ein Analgetikum, das bei starken und mittelstarken Schmerzen wirksam ist.

Die am Markt befindlichen peroralen Tramadol-Retard-Arzneiformen sind auf der Basis von Tablettenzubereitungen formuliert, die die bekannten Nachteile der Single Unit Dosage Form gegenüber der Multiple Unit Dosage Form besitzen. Single Units sind Einzelarzneiformen, die beispielsweise unzerfallen den Magen-Darm-Trakt passieren (Gerüsttabletten), durch Abbau immer kleiner werden (Erosionstabletten) oder deren Wirkstoff erst im Darm freigesetzt wird (magensaft-resistente Tabletten).
Multiple Units sind Arzneiformen, die nach Einnahme in eine Vielzahl von Untereinheiten zerfallen und bei denen die Untereinheiten Träger der Eigenschaften der Arzneiform sind. Bei den Single Unit-Retardarzneimitteln können erhebliche Schwankungen in der Magenverweildauer auftreten, was zu einer ungleichmäßigen Passage durch den Magen-Darm-Trakt und damit zu unterschiedlichen Blutspiegelwerten führen kann. Auch lokale Irritationen sind bei Multiple Unit Arzneiformen weniger zu befürchten als bei Single Unit Arzneiformen.
Das gleiche gilt für die Gefahr des "dose dumping", der zu schnellen Freisetzung des Wirkstoffes aus Retardarzneimitteln (H. Blume, "Biopharmazeutische Aspekte von Multiple Unit Dosage Forms; ein Vergleich mit Single Units", Druckschrift der Capsugel, Basel (Schweiz), über ein Symposium vom November 1988 in Hamburg).

Auch die im EP 147 780, EP 624 366, EP 654 263, EP 731 694 und DE 43 29 794 vorzugsweise beschriebenen Tramadol-Arzneiformen stellen derartige Single Unit Arzneiformen dar, die die vorgenannten Nachteile aufweisen können.
Pharmazeutische Formulierungen mit Retardüberzügen werden beispielsweise im EP 147 780 in Form von Tabletten und Granulaten beschieben, die einen Wirkstoffkern enthalten und mit einem Film aus Polyvinylalkohol überzogen sind.
Im EP 624 366 werden orale Tramadolpräparate mit verzögerter Freisetzung aufgezeigt, die vorzugsweise in Form von Tabletten verabreicht werden können. Der Wirkstoff ist hierbei insbesondere in einer Retardmatrix aus hydrophilen oder hydrophoben Polymeren, langkettigen Fettsäuren oder Fettalkoholen und einem oder mehreren Polyalkylenglykolen eingebettet.
Alternativ dazu werden filmbeschichtete Spheroide beschrieben. Der Wirkstoff ist in einem "spheronisierenden" Material, wie mikrokristalline Cellulose eingebettet und mit einem kontrolliert freisetzenden Film überzogen.
In EP 731 694 (WO95/14460) werden opioide Retardformulierungen, beispielsweise Pellets beschrieben, die einen analgetischen Effekt nach oraler Applikation für mindestens 24 Stunden gewährleisten.
Die Pellets bestehen aus einem wirkstoffhaltigen Kern, der mit einem zur retardierenden Freisetzung geeigneten Polymerfilm umhüllt ist. Der Film enthält neben den Polymeren insbesondere eine säurelösliche Verbindung sowie einen Weichmacher.
Der Einsatz von Weichmachern kann sich jedoch sehr nachteilig auswirken.
Bekannte Nachteile der Weichmacher können darin bestehen, daß diese aus der Membran auswandern, was die Wirkstofffreisetzung im Laufe der Lagerung beeinflussen kann.

Im EP 147 780 wird auf Seite 2 beschrieben, daß es auch zu einer chemischen Interaktion zwischen Weichmacher und dem Wirkstoff kommen kann, die zu einer Verringerung der Haltbarkeit des Produktes führt.
In Sucker, Fuchs, Speiser "Pharmazeutische Technologie " Thieme Verlag Stuttgart, 1978, wird auch ausgeführt, daß der Weichmacher neben seiner eigentlichen filmverbessernden Eigenschaft die Wasserdampfdurchlässigkeit und den Zerfall beeinflußt. Ferner führt der zwar niedrige doch merkliche Dampfdruck zu einem Verdunsten der Weichmacher verbunden mit der Änderung der physikalischen Eigenschaften, wie der Lösegeschwindigkeit des Films.

Die US-A-5,474,786 offenbart eine Mehrschicht-Retard-Formulierung für Tramadol, die in Form von Pellets vorliegen dann. Diese Formulierung besteht aus einem mit Tramadol überzogenen Startermaterial, welches mit sieben definierten Schichten überzogen ist. Die Schichten 1, 3, 5 und 7 enthalten ein wasserlösliches, filmbildendes Polymer und vorzugsweise auch einen Weichmacher. Die Schichten 2 und 6 sind Retardmembrane, die aus einer Polymerdispersion gebildet werden, und welche vorzugsweise auch einen Weichmacher enthalten. Die Schicht 4 ist eine weitere Wirkstoffschicht.

Die WO-A-95 14460 offenbart eine Opiat-enthaltende Retardformulierung, die in Form von Pellets vorliegen kann. Die Retardpellets bestehen aus einem wirkstoffhaltigen Kern, der mit einem geeigneten, die Freisetzung verzögernden Stoff überzogen ist. Unter anderem erwähnt dieses Dokument auch Tramadol. Als Stoffe, die die Freisetzung verzögern können, werden unter anderem acrylische Polymere, Alkylcellulosen oder Schellacke erwähnt. Solchen hydrophobischen Polymeren wird vorzugsweise auch ein Weichmacher zugesetzt. Insbesondere bei der Verwendung von Ethylcellulose ist ein Zusatz eines Weichmachers notwendig, um einen flexiblen Film für die Umhüllung unter normalen Umhüllungsbedingungen zu erhalten.

Bisher ist noch keine Tramadol-Pellet-Form auf dem Markt. Pelletformulierungen zeichnen sich wegen des Vorliegens vieler, oft einige Hunderte betragende Untereinheiten pro therapeutischer Dosis durch eine große Oberfläche aus. Diese kann bei leicht löslichen Stoffen wie Tramadol-Hydrochlorid zu einem Bedarf an Retardmaterial führen, der den Gehalt an Wirkstoff in der Pelletformulierung in unerwünschter Weise deutlich reduziert.
Dies ist besonders unerwünscht bei Wirkstoffen, die in höherer Dosierung verabreicht werden, weil die erforderliche Pelletmenge die Verwendung einer größeren Hartgelatine-Kapsel erfordert. Das ist therapeutisch unerwünscht, weil es für den Patienten unangenehm ist.

Der Erfindung liegt somit die Aufgabe zugrunde, Tramadol Multiple Unit Formulierungen in einer für den Patienten besonders annehmbaren und einfachen Ausführung bereitzustellen, die die Nachteile von Single Units vermeiden und damit eine verzögerte, gut reproduzierbare Freisetzung gewährleisten.

Gegenstand der Erfindung sind dementsprechend Multiple Unit Formulierenden, enthaltend einzelne gleich oder unterschiedlich retardierte Pellets, die aus einem mit Tgramadol oder dessen physiologisch verträglichen Salzen überzogenen Startermaterial bestehen, welches mit einer oder mehreren Membranschichten zur kontrollierten Freisetzung, bestehend aus einer Substanz aus der Gruppe der Ethylcellulosen verschiedenen Ethylierungsgrades und verschiedener Kettenlängen oder aus der Gruppe der Schellacke ohne Zusatz eines Weichmachers überzogen ist, wobei die aktive Substanz aus den Pellets verzögert im Magen-Darm-Trakt freigesetzt wird.

Die die Wirkstoff-Freisetzung aus Pellets kontrollierenden Mechanismen sind im wesentlichen durch folgende Funktionsprinzipien zu beschreiben:
Eine erste Möglichkeit zur Formulierung von Retardpellets besteht in der Steuerung der Freisetzung durch eine äußere Membran.
Ein anderer Weg, eine geeignete verzögerte Freisetzung zu erzielen, besteht darin, von einer äußeren Membran freie Pellets aufzubauen, deren Freisetzung durch eine kontrolliert freisetzende Matrix erfolgt.
Eine dritte Möglichkeit schließlich besteht in der Kombination beider genannten Maßnahmen, also der Kombination von Matrix- und Membransteuerung, um die gewünschten Eigenschaften zu erzielen.

Tramadolhydrochlorid zeichnet sich durch Löslichkeitseigenschaften aus, die bei den erfindungsgemäßen Multiple Unit Formulierungen zur Formulierung typischer membrangesteuerter Diffusionspellets ausgenutzt werden.

Die in den Multiple Unit Formulierungen enthaltenen Pellets bestehen aus einem wirkstoffhaltigen Kern, der mit einer oder mehreren Membranschichten überzogen ist, die der Steuerung der Wirkstoff-Freisetzung dienen.

Auf die Membran kann mit einem geeigneten Bindemittel eine schnellzerfallende oder schnellösliche Wirkstoffschicht (Initialdosis) aufgebracht sein, die zur Steuerung der Freisetzung unmittelbar nach der Applikation geeignet ist.

Der Wirkstoff kann in unveränderter Form oder auch in Form von Mischungen, die die Verarbeitbarkeit des Wirkstoffes positiv beeinflußen, verarbeitet werden.
Beispielsweise können Mischungen mit kolloidem Siliciumdioxid wie Aerosil200, Saccharose oder anderen geeigneten Stoffen verwendet werden.

Der Pellet-Kern selbst besteht aus einem inneren, inerten Startermaterial, auf das mit einer geeigneten Lösung der Wirkstoff aufgebracht ist.
Als Startermaterial zur Pelletherstellung können beispielsweise Saccharose-Kristalle oder auch Saccharose-Maisstärke-Pellets (Nonpareilles, Neutralpellets, Sugar Spheres USP 23/NF 18) eingesetzt werden.

Der Wirkstoff kann aufgrund seiner guten Löslichkeit in Wasser, niederen Alkoholen wie Ethanol, Isopropanol und Alkohol-Wasser-Mischungen oder Aceton in Lösungen dieser Lösungsmittel auf das Startermaterial aufgebracht werden.
Der Prozess kann beschleunigt werden, indem der Wirkstoff in pulvriger Form und Wirkstoff in gelöster Form oder mit Lösungsmitteln allein gleichzeitig aufgebracht werden.

Eine andere, bevorzugte Variante dieses Herstellungsteilschrittes ist es, den Wirkstoff in Pulverform mit einer geeigneten Bindemittel-Lösung auf das Startermaterial aufzutragen.
Dieses Aufbringen des Wirkstoffes erfolgt schichtweise dadurch, daß nach mehr oder weniger großen Auftragsmengen der Prozess mehr oder weniger lange unterbrochen wird.

Von der Bindemittel-Lösung, die ein Bindemittel oder eine Kombination von mehreren Bindemitteln enthalten kann, wird nur soviel eingesetzt, daß der Wirkstoff auf dem Startermaterial fixiert wird. Die Wirkstoffschicht besteht damit überwiegend aus dem Wirkstoff selbst und nur zu 10 % oder weniger aus einem oder mehreren Bindemitteln.

Als Bindemittel können Polyvinylpyrrolidone, wie PVP 25, hydrophile Celluloseether, wie Hydropropylmethylcellulose, Ethylcellulosen verschiedenen Ethylierungsgrades und verschiedener Kettenlangen, Schellacke, Copolymerisate mit anionischem Charakter auf der Basis von Methacrylsäure und Methylmethacrylat oder Ethylacrylat entsprechend den im USP 23/NF 18 beschriebenen Stoffen Methacrylic Acid Copolymer Type A, B und C, wie Eudragit L®, Eudragit S® und Eudragit L 100-55® oder Ammonio Methacrylate Copolymer Type A und B, wie Eudragit RL® und Eudragit RS® , Celluloseacetatphthalat und Hydroxypropylmethylcelluloseacetatphthalat eingesetzt werden.

Als Lösungsmittel für die Bindemittel und/oder für die die Freisetzung verzögernden Stoffe eignen sich insbesondere Wasser, niedere Alkohole wie Ethanol, Isopropanol, Alkohol-Wasser-Mischungen oder Aceton.

Die Stoffe zur Verzögerung der Freisetzung können getrennt oder in Kombination miteinander verarbeitet werden. Erfindungsgemäß werden Substanzen aus der Gruppe der Ethylcellulosen und/oder aus der Gruppe der Schellacke eingesetzt.
Beispielsweise können Kombinationen von Ethylcellulose mit Schellack im Verhältnis 1: 9 bis 9 : 1 verwendet werden. Ebenso sind Kombinationen von Ethylcellulose mit Eudragit S® geeignet.

Eine besonders bevorzugte Ausfuhrungsform des erfindungs- gemaßen Gegenstandes besteht darin, daß sowohl als Bindemittel als auch die Freisetzung verzogerndene Stoffe Gemische von Ethylcellulose und Schellacken eingesetzt werden.
Durch die Verwendung der gleichen Polymere für den Wirkstoffauftrag auf das Startermaterial wie auch für die Retardierung erhält man besonders einfach zusammengesetzte Tramadol-Pellets, die leicht herzustellen sind, wenig verschiedene Hilfsstoffe enthalten und damit für den Patienten von großem Vorteil sind.
Als weitere Bestandteile können die erfindungsgemäßen Multiple Unit Formulierungen pharmazeutisch gebräuchliche Hilfsstoffe wie Trenn- und Fließregulierungsmittel, beispielsweise hochdisperses Siliciumdioxid, Talkum und Magnesiumstearat enthalten.

Die Pellets können gegebenenfalls unterschiedlich retardiert sein, bedingt durch unterschiedliche Schichtdicken der Membran oder auch durch den Einsatz unterschiedlicher Retardierungsmittel. Damit ist eine weitere Möglichkeit zur Steuerung der Freisetzung des Wirkstoffes gegeben.
Diese gleich oder verschieden retardierte Pellets enthaltenden Multiple Unit Formulierungen passieren im Gegensatz zu den Single Unit Formulierungen fortlaufend den Pylorus, selbst in dessen geschlossenem Zustand, und verteilen sich über den gesamten Magen-Darm-Bereich. Das führt zu einer recht gleichmäßigen Passage durch den Gastrointestinaltrakt.

Die Herstellung der Pellets erfolgt nach üblichen Methoden (Pharmaceutical Pelletization Technology, edited by Isaac Ghebre-Sellassie, Marcel Dekker Verlag, New York and Basel, 1989).

Ein bevorzugtes Verfahren zur Herstellung wird folgendermaßen durchgeführt:

Im Dragierkessel oder einer anderen geeigneten Apparatur werden Starterkerne vorgelegt, zu diesen wird der Wirkstoff oder eine Wirkstoffmischung kontinuierlich oder diskontinuierlich zugegeben und mit einer Lösung von Bindemitteln auf dem Startermaterial fixiert. Mit einer gleichen oder anderen Lösung von Bindemitteln und/oder die Freisetzung regulierenden Stoffen wird nach der Beladung des Startermaterials mit Wirkstoff eine Membran aufgebracht, bis die Freisetzung den geforderten. Normen entspricht. Die wirkstoffhaltigen, den Wirkstoff retardiert freisetzenden Pellets können mit zusätzlichem Wirkstoff, der nicht retardiert und als Initialdosis freigesetzt wird, durch verschiedene, dem Fachmann bekannte Verfahren, beispielsweise Dragieren oder Aufsprühen aus Lösungen umhüllt sein.
Die Umhüllung kann außer im Dragierkessel auch in der Wirbelschicht erfolgen.

Durch Trocknungsvorgänge während und/oder nach den einzelnen Arbeitsschritten werden akzeptable Grenzwerte für die verwendeten Lösungsmittel eingestellt.

Im WO 95/14460 wird ein kompliziertes Verfahren zur Herstellung von Pellets mit einer Umhüllung aus Ethylcellulose und einem Weichmacher aufgezeigt.
In dem sogenannten "Curing"-Verfahren werden die Pellets statt der üblichen Nachtrocknung einem technisch schwer durchführbaren Temperatur- und Feuchtigkeitsstreß ausgesetzt, der beispielsweise bei 60 Grad Celcius und 80 % relative Luftfeuchtigkeit durchgeführt wird. Dieses Verfahren ist durch die extremen Bedingungen nicht für jeden Wirkstoff geeignet. Tramadol-Pellets würden aufgrund der Feuchtigkeitsempfindlichkeit von Tramadol unter diesen Bedingungen zerfallen.

Die erfindungsgemäßen Tramadol-Pellets besitzen einen Teilchendurchmesser von 0,4 - 3,0 mm, vorzugsweise liegen die Durchmesser im Bereich von 0,6 - 1,6 mm.

Die gegebenenfalls unterschiedlich retardierten Pellets können zur peroralen Verabreichung vorzugsweise in Gelatinekapseln abgefüllt oder mit geeigneten Hilfsstoffen zu Tabletten verpreßt werden.
Die Abfüllung in Kapseln hat den großen Vorteil, daß Patienten mit Problemen, Kapseln oder Tabletten zu schlucken, die Einnahme wesentlich erleichtert werden kann. Durch das Öffnen der Kapseln können die Retardpellets entnommen und mit Flüssigkeit oder Speisebrei geschluckt werden. Im übrigen können die erfindungsgemäßen Retardpellets auch über eine Magen-oder Duodenalsonde verabreicht werden.

Der Gehalt des verzögert freizusetzenden Wirkstoffes in den Multiple Unit Formulierungen liegt zwischen 30 und 85 Gew.-%. Bevorzugt werden Multiple Unit Formulierungen mit einem Gehalt an verzögert freizusetzendem Wirkstoff zwischen 50 und 75 Gew.-%.
Der Gehalt an pharmazeutisch akzeptablen, die Freisetzung verzögernden Stoffen beträgt 2 bis 40 Gew.-%.

Je nach Ausführung der erfindungsgemäßen Formulierungen ist es möglich, die für den erwünschten therapeutischen Effekt erforderliche Tagesdosis durch einmalige (24-Stunden-Zubereitung) oder zweimalige Gabe(12 Stunden-Zubereitung) zu verabreichen. Eine Beschränkung auf bestimmte Dosierungen und auf einen bestimmten Dosisbereich ist nicht gegeben. Die Wirkstoffmenge und die Wirkstofffreisetzung kann allen therapeutischen Bedürfnissen angepaßt werden.
In Bioverfügbarkeitsstudien wurde belegt, daß nach der Applikation der erfindungsgemäßen Formulierung in Form von Kapseln , die beispielsweise 50 bis 200 mg Tramadol-hydrochlorid enthalten, alle biopharmazeutischen Parameter den nach dem Stand der Wissenschaft erforderlichen Anforderungen an ein Tramadol-Retardpräparat entsprechen.

### Wirkstofffreisetzung

Ein geeignetes in-vitro-Modell zur analytischen Beurteilung der Wirkstoff-Freisetzung sollte den physiologischen pH-Verlauf des Magen-Darm-Traktes nachvollziehen und Testflüssigkeiten aufweisen, die vom sauren über den schwach sauren bis zum schwach alkalischen pH-Wert reichen.
Das Hauptkriterium zur in-vitro-Qualitätsbeurteilung der Wirkstoff-Freisetzung von Retardpräparaten vom Multiple Unit-Typ mit Wirkstoffen vom Löslichkeitsverhalten des Tramadolhydrochlorid ist der Nachweis einer ausreichenden Retardierung im sauren pH-Bereich und neben dem erwünschten Retardierungsgrad insbesondere eine vollständige Wirkstofffreisetzung im schwach sauren bis schwach alkalischen Bereich.
Ein für diese Art von Untersuchungen geeignetes Gerät beschreibt das Amerikanische Arzneibuch (USP 23) als "Apparatus 3" auf den Seiten 1793 und 3012, eine eingehendere Beschreibung des Gerätes findet sich im Journal of Pharmaceutical Sciences, Band 80 (1991), Seite 991 - 994.

Die Erfindung soll anhand von Beispielen, die keine Beschränkungen des erfindungsgemäßen Gegenstandes bedeuten sollen, näher erläutert werden.

### Beispiel 1

### Herstellung der wirkstoffhaltigen Kerne

Auf 1000 g Neutralpellets geeigneter Größe (z. B. mit einem Durchmesser zwischen 0,5 und 0,6 mm) wurden mit ca. 1150 g einer 20 %igen Lösung von Ethylcellulose/Schellack (2 : 8) in einem Ethanol-Wasser-Gemisch ca. 96 % [V/V] 4020 g Tramadolhydrochlorid-Aerosil® 200 - Gemisch im Drageekessel aufgebracht. Die erhaltenen Kerne wurden anschließend getrocknet und gesiebt (0,8 - 1,4 mm).

### Aufbringung der Membran

Auf 5,25 kg der so hergestellten wirkstoffhaltigen Kerne wurden Membranen aufgebracht, indem 390g einer 20 %igen Lösung von Ethylcellulose/Schellack (2 : 8) in einem Ethanol-Wasser-Gemisch ca.96 %[V/V] aufgegeben wurden. Als Trennmittel wurden 780 g Talkum eingepudert.

| Rezeptur | |
|---|---|
| | Gewichtsteile (%) |
| Tramadolhydrochlorid | 65,5 |
| Neutralpellets | 16,4 |
| Ethylcellulose | 1,0 |
| Schellack | 4,0 |
| Aerosil 200 | 0,3 |
| Talkum | 12,8 |
| Ethanol-Wasser-Gemisch ca. 96 % [V/V] | q.s. |

Die in-vitro-Freisetzung von Tramadolhydrochlorid aus den Retardpellets gemäß Beispiel 1 wurde nach USP 23/NF 18 in Apparat 3 bestimmt. Die Temperatur des Freisetzungsmediums betrug 37° C, die Hubzahl der Probenröhren 20 Hübe/Minute und die Menge Testlösung pro Untersuchungsintervall 175 ml.

Die Untersuchung wurde mit Testlösung pH 1.5 begonnen, nach der ersten Stunde wechselten die Röhren mit den Proben in jeweils 175 ml Testlösung pH 4.5, nach der 2. Stunde in eine Testlösung pH 6.9, nach der 4. Stunde in eine neue Testlösung pH 6.9, nach der 6. Stunde in Testlösung pH 7.2 und nach der 8. Stunde in eine Testlösung pH 7.5. Die zu den vorgenannten Zeitpunkten im Lösungsmedium befindliche freigesetzte Wirkstoffmenge wurde spektralphotometrisch bestimmt. Es wurden folgende Freisetzungswerte ermittelt:

| Zeit in Stunden | Freigesetzter Anteil in Gew.- % |
|---|---|
| 1 | 34 |
| 2 | 51 |
| 4 | 65 |
| 6 | 75 |
| 8 | 84 |
| 12 | 98 |

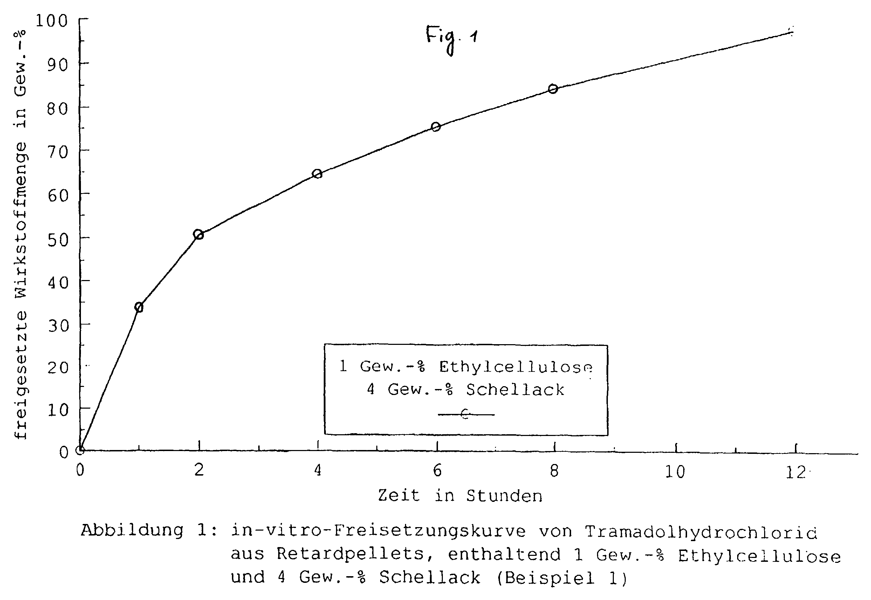

Die in-vitro-Freisetzungskurve der Retardpellets gemäß Beispiel 1 ist in der Abbildung 1 dargestellt.

### Beispiel 2

Analog Beispiel 1 wurden Retardpellets folgender Rezeptur hergestellt:

| | Gewichtsteile (%) |
|---|---|
| Tramadolhydrochlorid | 50,0 |
| Neutralpellets | 12,2 |
| Ethylcellulose | 8,4 |
| Eudragit S ® | 0,9 |
| Aerosil 200 ® | 0,2 |
| Talkum | 28,3 |
| Aceton | q.s. |

Die in-vitro-Freisetzungsuntersuchung wurde analog Beispiel 1 durchgeführt.
Es wurden folgende Freisetzungswerte ermittelt:

| Zeit in Stunden | Freigesetzter Anteil in Gew.- % |
|---|---|
| 1 | 30 |
| 2 | 51 |
| 4 | 70 |
| 6 | 81 |
| 8 | 88 |
| 12 | 94 |

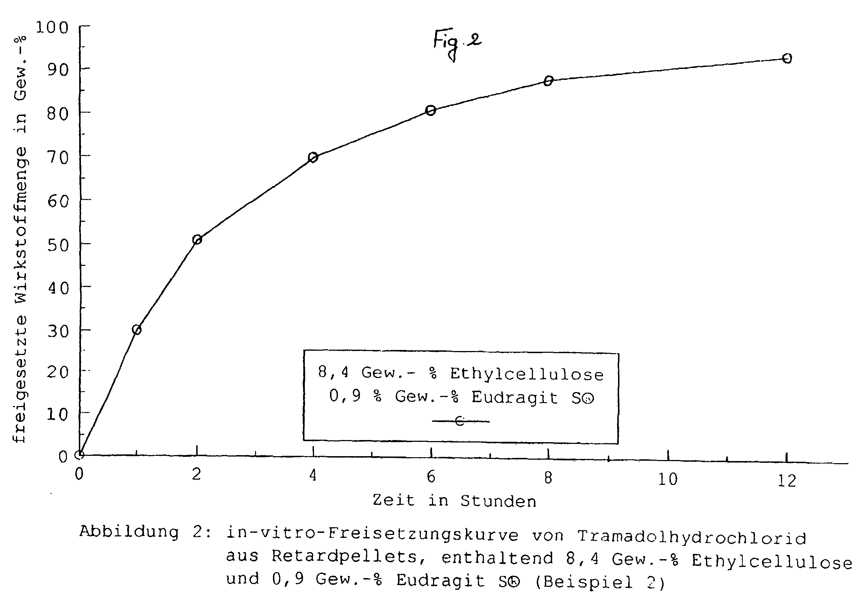

Die in-vitro-Freisetzungskurve der Retardpellets gemäß Beispiel 2 ist in der Abbildung 2 dargestellt.

### Beispiel 3

Analog Beispiel 1 wurden Retardpellets folgender Rezeptur hergestellt:

| | Gewichtsteile (%) |
|---|---|
| Tramadolhydrochlorid | 40,3 |
| Neutralpellets | 10,1 |
| Ethylcellulose | 6,7 |
| Aerosil 200® | 0,2 |
| Talkum | 42,7 |
| Ethanol-Wasser-Gemisch ca. 96% [V/V] | q.s. |

Die in-vitro-Freisetzungsuntersuchung wurde analog Beispiel 1 durchgeführt.
Es wurden folgende Freisetzungswerte ermittelt:

| Zeit in Stunden | Freigesetzter Anteil in Gew.- % |
|---|---|
| 1 | 34 |
| 2 | 59 |
| 4 | 81 |
| 6 | 89 |
| 8 | 92 |
| 12 | 93 |

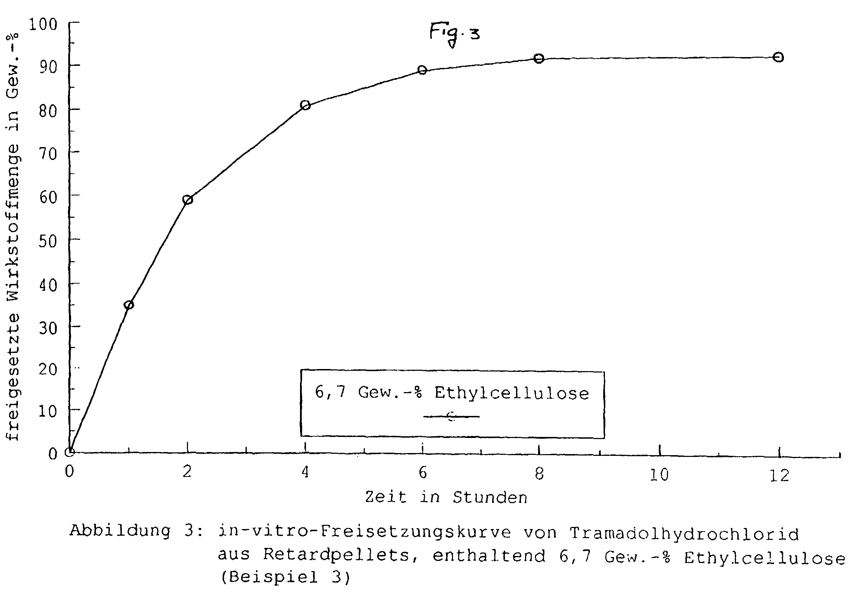

Die in-vitro-Freisetzungskurve der Retardpellets gemäß Beispiel 3 ist in der Abbildung 3 dargestellt.

### Beispiel 4

Analog Beispiel 1 wurden Retardpellets folgender Rezeptur hergestellt:

| | Gewichtsteile (%) |
|---|---|
| Tramadolhydrochlorid | 59,7 |
| Neutralpellets | 14,9 |
| Ethylcellulose | 0,7 |
| Schellack | 4,7 |
| Aerosil 200® | 0,3 |
| Talkum | 19,7 |
| Ethanol-Wasser-Gemisch ca. 96% [V/V] | q.s. |

Die in-vitro-Freisetzungsuntersuchung wurde analog Beispiel 1 durchgeführt.
Es wurden folgende Freisetzungswerte ermittelt:

| Zeit in Stunden | Freigesetzter Anteil in Gew.- % |
|---|---|
| 1 | 35 |
| 2 | 52 |
| 4 | 72 |
| 6 | 84 |
| 8 | 95 |
| 12 | 100 |

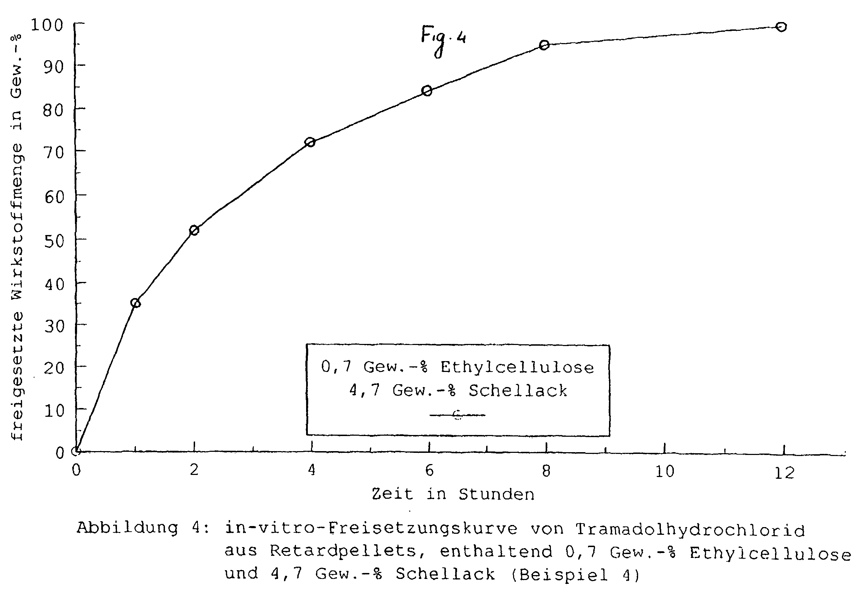

Die in-vitro-Freisetzungskurve der Retardpellets gemäß Beispiel 4 ist in der Abbildung 4 dargestellt.

### Beispiel 5

Analog Beispiel 1 wurden Retardpellets folgender Rezeptur hergestellt:

| | Gewichtsteile (%) |
|---|---|
| Tramadolhydrochlorid | 48,8 |
| Neutralpellets | 12,2 |
| Ethylcellulose | 0,6 |
| Schellack | 2,3 |
| Eudragit RS® | 2,2 |
| Aerosil 200® | 0,2 |
| Talkum | 33,7 |
| Ethanol-Wasser-Gemisch ca. 96% [V/V] | q.s. |

Die in-vitro-Freisetzungsuntersuchung wurde analog Beispiel 1 durchgeführt.
Es wurden folgende Freisetzungswerte ermittelt:

| Zeit in Stunden | Freigesetzter Anteil in Gew.- % |
|---|---|
| 1 | 31 |
| 2 | 58 |
| 4 | 76 |
| 6 | 83 |
| 8 | 86 |
| 12 | 89 |

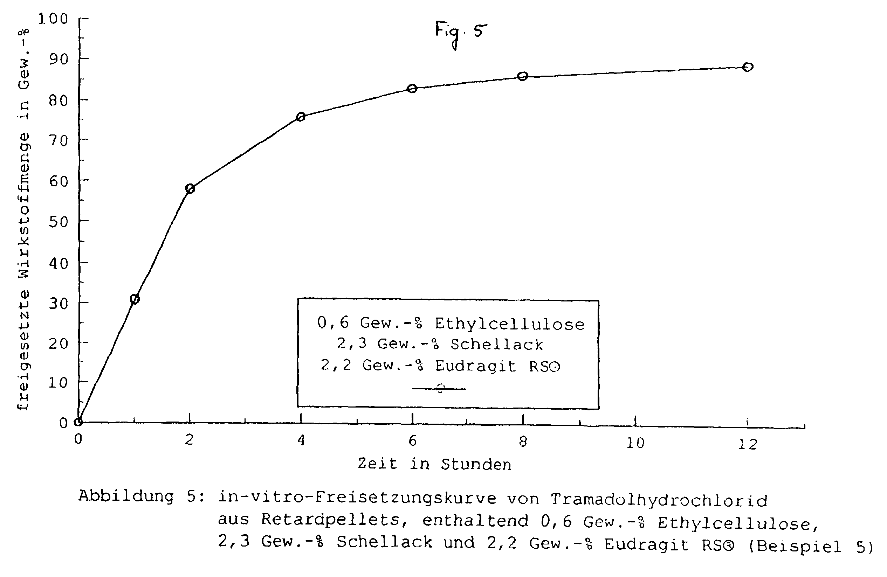

Die in-vitro-Freisetzungskurve der Retardpellets gemäß Beispiel 5 ist in der Abbildung 5 dargestellt.

### Beispiel 6

Analog Beispiel 1 wurden Retardpellets folgender Rezeptur hergestellt:

| | Gewichtsteile (%) |
|---|---|
| Tramadolhydrochlorid | 64,4 |
| Neutralpellets | 16,1 |
| Ethylcellulose | 3,5 |
| Schellack | 2,3 |
| Aerosil 200® | 0,3 |
| Talkum | 13,4 |
| Ethanol-Wasser-Gemisch ca.96% [v/v] | q.s. |

Die in-vitro-Freisetzungsuntersuchung wurde analog Beispiel 1 durchgeführt.
Es wurden folgende Freisetzungswerte ermittelt:

| Zeit in Stunden | Freigesetzter Anteil in Gew.- % |
|---|---|
| 1 | 39 |
| 2 | 57 |
| 4 | 70 |
| 6 | 78 |
| 8 | 84 |
| 12 | 93 |

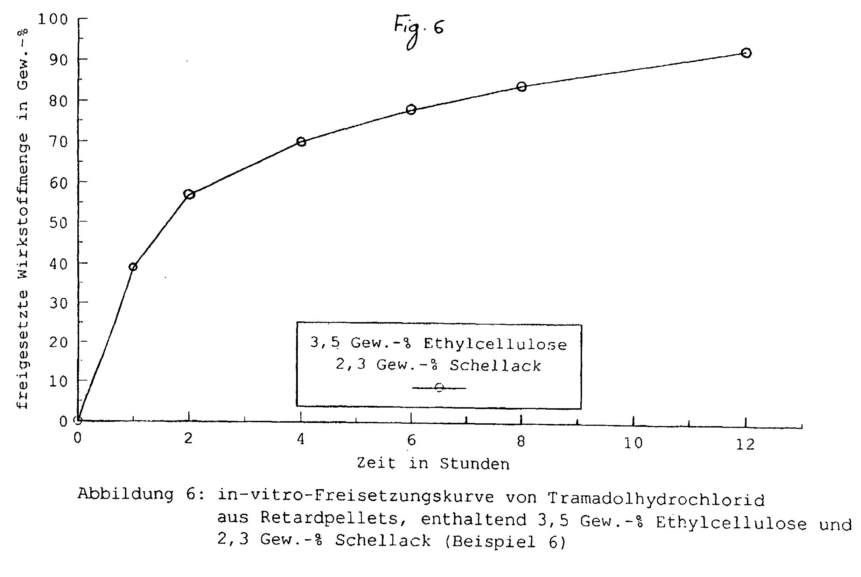

Die in-vitro-Freisetzungskurve der Retardpellets gemäß Beispiel 6 ist in der Abbildung 6 dargestellt.

### Beispiel 7

Analog Beispiel 1 wurden Retardpellets folgender Rezeptur hergestellt:

| | Gewichtsteile (%) |
|---|---|
| Tramadolhydrochlorid | 58,9 |
| Neutralpellets | 14,8 |
| Ethylcellulose | 0,6 |
| Schellack | 4,9 |
| Aerosil 200® | 0,3 |
| Talkum | 20,5 |
| Ethanol-Wasser-Gemisch ca.96% [v/v] | q.s. |

Die in-vitro-Freisetzungsuntersuchung wurde analog Beispiel 1 durchgeführt.

Es wurden folgende Freisetzungswerte ermittelt:

| Zeit in Stunden | Freigesetzter Anteil in Gew.- % |
|---|---|
| 1 | 3 |
| 2 | 11 |
| 6 | 47 |
| 10 | 69 |
| 14 | 82 |
| 22 | 98 |

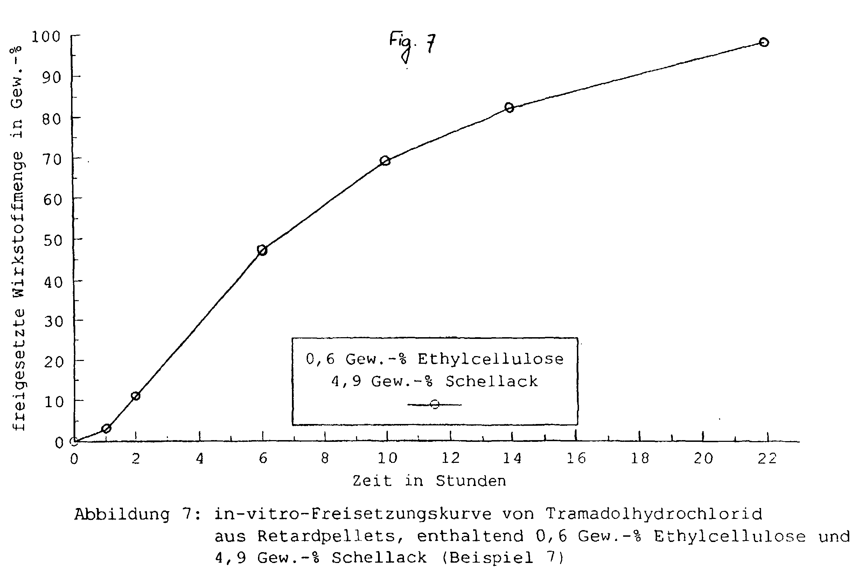

Die in-vitro-Freisetzungskurve der Retardpellets gemäß Beispiel 7 sind in Abbildung 7 dargestellt.

## Patentansprüche

1. , Pharmazeutische Multiple Unit Formulierungen enthaltend einzelne gleich oder unterschiedlich retardierte Pellets bestehend aus einem mit Tramadol oder dessen physiologisch verträglichen Salzen überzogenen Startermaterial, welches mit einer oder mehreren Membranschichten zur kontrollierten Freisetzung bestehend aus einer Substanz aus der Gruppe der Ethylcellulosen verschiedenen Ethylierungsgrades und verschiedener Kettenlängen und/oder aus der Gruppe der Schellacke ohne Zusatz eines Weichmachers überzogen ist.

2. Pharmazeutische Formulierungen nach Anspruch 1 **dadurch gekennzeichnet, daß** der verzögert freizusetzende Wirkstoffgehalt zwischen 30 und 85 Gew.-% und der Gehalt an pharmazeutisch akzeptablen, die Freisetzung verzögernden Stoffen zwischen 2 und 40 Gew.-% beträgt.

3. Pharmazeutische Formulierungen nach Anspruch 1 **dadurch gekennzeichnet, daß** der verzögert freizusetzende Wirkstoffgehalt zwischen 50 und 75 Gew.-% und der Gehalt an pharmazeutisch akzeptablen, die Freisetzung verzögernden Stoffen zwischen 2 und 40 Gew.-% beträgt.

4. Pharmazeutische Formulierungen nach Anspruch 1 **dadurch gekennzeichnet, daß** als Startermaterial für die Retardpellets Saccharose-Kristalle oder Nonpareilles (Neutralpellets, Sugar Spheres) eingesetzt werden.

5. Pharmazeutische Formulierungen nach Anspruch 1 **dadurch gekennzeichnet, daß** das Verhältnis zwischen einer Substanz aus der Gruppe der Ethylcellulosen und aus der Gruppe der Schellacke vorzugsweise 1:9 bis 9:1 beträgt.

6. Pharmazeutische Formulierungen nach Anspruch 1, **dadurch gekennzeichnet, daß** Tramadol oder dessen physiologisch verträgliches Salz als Lösung oder in Pulverform mit einer Wirkstofflösung oder mit einem geeigneten Lösungsmittel oder in einer geeigneten Bindemittellösung auf das Startermaterial aufgebracht wird.

7. Pharmazeutische Formulierung nach Anspruch 6 **dadurch gekennzeichnet, daß** als Lösungsmittel niedere Alkohole wie Ethanol, Isopropanol, Alkohol-Wasser-Mischungen oder Aceton eingesetzt werden.

8. Pharmazeutische Formulierungen nach Anspruch 6 **dadurch gekennzeichnet, daß** als Bindemittel Ethylcellulosen verschiedenen Ethylierungsgrades und verschie-dener Kettenlängen und/oder Schellacke eingesetzt werden.

9. Pharmazeutische Formulierungen nach den Ansprüchen 6 - 8 **dadurch gekennzeichnet, daß** Tramadol oder deren physiologisch verträglichen Salze bevorzugt in Pulverform mit einer Bindemittellösung bestehend aus Ethylcellulose und Schellack in einer Ethanol-Wasser-Mischung auf das Startermaterial aufgebracht wird.

10. Pharmazeutische Formulierungen nach Anspruch 1 **dadurch gekennzeichnet, daß** als weitere Hilfsstoffe Trenn- und Fließmittel wie Siliciumdioxid oder Talkum enthalten sein können.

11. Pharmazeutische Formulierungen nach Anspruch 1 **dadurch gekennzeichnet, daß** der Teilchendurchmesser der Retardpellets 0,4 - 3,0 mm, vorzugsweise 0,6 - 1,6 mm beträgt.

12. Pharmazeutische Formulierungen nach Anspruch 1 in Form von Kapseln oder Tabletten.

13. Pharmazeutische Formulierungen nach Anspruch 12 **dadurch gekennzeichnet, daß** die Retardpellets aus den Kapseln entnommen und gegebenenfalls separat verabreicht werden können.

14. Pharmazeutische Formulierungen nach Anspruch 1 zur einmaligen (24-Stunden-Zubereitung) oder zweimaligen Dosierung (12-Stunden-Zubereitung).

15. Verfahren zur Herstellung von Multiple Unit Formulierungen gemäß Anspruch 1 **dadurch gekennzeichnet, daß** der Wirkstoff oder eine Wirkstoffmischung auf ein Startermaterial mit Hilfe einer alkoholischen, alkoholisch-wässrigen oder acetonigen Lösung oder Bindemittellösung aufgebracht, danach mit einer Lösung bestehend aus Ethylcellulosen und/oder Schellacken, gegebenenfalls unter Verwendung von Trennmitteln, zum Aufbau einer geeigneten Membran behandelt wird und die erhaltenen Pellets anschließend in Kapseln abgefüllt oder zu Tabletten verpreßt werden.

## Claims

1. Pharmaceutical multiple unit formulations comprising individual pellets which are delayed to an identical or different extent consisting of a starter material coated with tramadol or its physiologically tolerable salts, which is coated with one or more membrane layers for controlled release consisting of a substance from the group of ethylcelluloses having various degrees of ethylation and various chain lengths and/or from the group of shellacs without addition of a softening agent.

2. Pharmaceutical formulations according to claim 1, **characterized in that** the active compound content which is to be released in delayed form is between 30 and 85% by weight and the content of pharmaceutically acceptable, release-delaying substances is between 2 and 40% by weight.

3. Pharmaceutical formulations according to claim 1, **characterized in that** the active compound content which is to be released in delayed form is between 50 and 75% by weight and the content of pharmaceutically acceptable, release-delaying substances is between 2 and 40% by weight.

4. Pharmaceutical formulations according to claim 1, **characterized in that** the starter materials employed for the delayed-release pellets are sucrose crystals or nonpareils (neutral pellets, sugar spheres).

5. Pharmaceutical formulations according to claim 1, **characterized in that** the ratio between a substance of the ethylcelluloses group and of the shellacs group is preferably 1 : 9 to 9 : 1.

6. Pharmaceutical formulations according to claim 1, **characterized in that** tramadol or its physiologically tolerable salts is/are applied to the starter material as a solution or in powder form with an active compound solution or a suitable solvent or a suitable binding agent solution.

7. Pharmaceutical formulation according to claim 6, **characterized in that** the solvents employed are lower alcohols such as ethanol, isopropanol, alcohol/water mixtures or acetone.

8. Pharmaceutical formulations according to claim 6, **characterized in that** ethylcelluloses having various degrees of ethylation and various chain lengths and/or shellacs are employed as binding agents.

9. Pharmaceutical formulations according to claims 6 - 8, **characterized in that** tramadol or its physiologically tolerable salts is/are preferably applied to the starter material in powder form using a binding agent solution consisting of ethylcellulose and shellac in an ethanol/water mixture.

10. Pharmaceutical formulations according to claim 1, **characterized in that** release agents and glidants such as silica or talc can be contained as further auxiliaries.

11. Pharmaceutical formulations according to claim 1, **characterized in that** the particle diameter of the delayed-release pellets is 0.4 - 3.0 mm, preferably 0.6 - 1.6 mm.

12. Pharmaceutical formulations according to claim 1 in the form of capsules or tablets.

13. Pharmaceutical formulations according to claim 12, **characterized in that** the delayed-release pellets can be removed from the capsules and, if desired, administered separately.

14. Pharmaceutical formulations according to claim 1 for single (24-hour preparation) or twice-repeated dosage (12-hour preparation).

15. Process for the preparation of multiple unit formulations according to claim 1, **characterized in that** the active compound or an active compound mixture is applied to a starter material with the aid of an alcoholic, alcoholic/aqueous or acetone solution or binding agent solution, then treated with a solution consisting of ethylcelluloses and/or shellacs, if desired using release agents, to construct a suitable membrane, and the pellets obtained are then dispensed in capsules or compressed to give tablets.

## Revendications

1. Formulations pharmaceutiques à unités multiples contenant des pastilles individuelles retardées de manière identique ou différente consistant en un matériau starter recouvert de tramadol ou de ses sels physiologiquement acceptables qui est recouvert d'une ou plusieurs couches de membrane pour la libération contrôlée consistant en une substance du groupe des éthylcelluloses de différents degrés d'éthylation et de différentes longueurs de chaîne et/ou du groupe des gommes-laques sans addition d'un plastifiant.

2. Formulations pharmaceutiques selon la revendication 1 **caractérisées en ce que** la teneur en principe actif à libérer de façon retardée est de 30 à 85 % en masse et la teneur en substances pharmaceutiquement acceptables retardant la libération est de 2 à 40 % en masse.

3. Formulations pharmaceutiques selon la revendication 1 **caractérisées en ce que** la teneur en principe actif à libérer de façon retardée est de 50 à 75 % en masse et la teneur en substances pharmaceutiquement acceptables retardant la libération est de 2 à 40 % en masse.

4. Formulations pharmaceutiques selon la revendication 1 **caractérisées en ce que** des cristaux de saccharose ou des non pareilles (pastilles neutres, sugar spheres) sont utilisés comme matériau starter pour les pastilles retard.

5. Formulations pharmaceutiques selon la revendication 1 **caractérisées en ce que** le rapport entre une substance du groupe des éthylcelluloses et une substance du groupe des gommes-laques est de préférence de 1:9 à 9:1.

6. Formulations pharmaceutiques selon la revendication 1 **caractérisées en ce que** le tramadol ou son sel physiologiquement acceptable est appliqué sur le matériau starter sous forme de solution ou sous forme de poudre avec une solution de principe actif ou avec un solvant approprié ou dans une solution de liant appropriée.

7. Formulation pharmaceutique selon la revendication 6 **caractérisée en ce que** des alcools inférieurs comme l'éthanol, l'isopropanol, des mélanges alcool-eau ou l'acétone sont utilisés comme solvant.

8. Formulations pharmaceutiques selon la revendication 6 **caractérisées en ce que** des éthylcelluloses de différents degrés d'éthylation et de différentes longueurs de chaîne et/ou des gommes-laques sont utilisées comme liant.

9. Formulations pharmaceutiques selon les revendications 6-8 **caractérisées en ce que** le tramadol, ou ses sels physiologiquement acceptables, est appliqué sur le matériau starter de préférence sous forme de poudre avec une solution de liant consistant en éthylcellulose et en gomme-laque dans un mélange éthanol-eau.

10. Formulations pharmaceutiques selon la revendication 1 **caractérisées en ce que** des agents de séparation et d'écoulement comme le dioxyde de silicium ou le talc peuvent être contenus à titre d'adjuvants supplémentaires.

11. Formulations pharmaceutiques selon la revendication 1 **caractérisées en ce que** le diamètre de particule des pastilles retard est 0,4 - 3,0 mm, de préférence 0,6 - 1,6 mm.

12. Formulations pharmaceutiques selon la revendication 1 sous forme de capsules ou de comprimés.

13. Formulations pharmaceutiques selon la revendication 12 **caractérisées en ce que** les pastilles retard sont prélevées sur les capsules et peuvent éventuellement être administrées séparément.

14. Formulations pharmaceutiques selon la revendication 1 pour le dosage unique (préparation de 24 heures) ou répété (préparation de 12 heures).

15. Procédé de préparation de formulations à unités multiples selon la revendication 1 **caractérisé en ce que** le principe actif ou un mélange de principes actifs est appliqué sur un matériau starter à l'aide d'une solution alcoolique, alcoolique-aqueuse ou acétonique ou d'une solution de liant, puis traité avec une solution consistant en éthylcelluloses et/ou gommes-laques, éventuellement au moyen d'agents de séparation, pour la formation d'une membrane appropriée et les pastilles obtenues sont ensuite introduites dans des capsules ou compressées en comprimés.
